(19) Europäisches Patentamt
European Patent Office
Office européen des brevets

(11) **EP 1 413 567 B1**

(12) **EUROPÄISCHE PATENTSCHRIFT**

(45) Veröffentlichungstag und Bekanntmachung des Hinweises auf die Patenterteilung:
**15.06.2016 Patentblatt 2016/24**

(51) Int Cl.:
*C07C 31/26* *(2006.01)* *C07C 29/78* *(2006.01)*

(21) Anmeldenummer: **03023425.6**

(22) Anmeldetag: **17.10.2003**

(54) **Verfahren zur Herstellung von sprühgetrocknetem Sorbitol**

Process for the preparation of spray-dried sorbitol

Procédé pour la préparation de sorbitol sec par atomisation

(84) Benannte Vertragsstaaten:
**AT BE BG CH CY CZ DE DK EE ES FI FR GB GR HU IE IT LI LU MC NL PT RO SE SI SK TR**

(30) Priorität: **22.10.2002 DE 10249336**

(43) Veröffentlichungstag der Anmeldung:
**28.04.2004 Patentblatt 2004/18**

(73) Patentinhaber: **DHW Deutsche Hydrierwerke GmbH**
**Rodleben**
**06862 Rodleben (DE)**

(72) Erfinder:
• **BALLNUS, Martina**
**06862 Rodleben (DE)**
• **KNOFE, Eberhard**
**06847 Dessau (DE)**
• **KONETZKE, Gerhard**
**06847 Dessau (DE)**
• **PESTER, H.-Jörgen**
**06844 Dessau (DE)**
• **SCHMIDT, Klaus**
**06862 Rodleben (DE)**
• **WEIDEMANN, Frank**
**06862 Meinsdorf (DE)**
• **WEIDEMANN, Reinhold**
**14827 Wiesenburg (DE)**
• **WÖHLING, H.-Joachim**
**39261 Zerbst (DE)**

(74) Vertreter: **Weidner Stern Jeschke**
**Patentanwälte Partnerschaft**
**Postfach 80 03 19**
**99029 Erfurt (DE)**

(56) Entgegenhaltungen:
**EP-A- 0 380 219 DE-A- 3 732 141**
**US-A- 4 605 794**

**Beschreibung**

Die Erfindung betrifft ein Verfahren zur Herstellung von sprühgetrocknetem Sorbitol.

Während für technische Anwendungsgebiete vorwiegend konzentrierte wässrige Sorbitollösungen eingesetzt werden, finden Sorbitole in fester Form vor allem in der Nahrungs- und Genussmittel- sowie in der pharmazeutischen Industrie Verwendung. Neben den durch die chemische Struktur der Sorbitole bedingten Eigenschaften sind beim Einsatz fester Produkte die äußere Gestalt der Partikel sowie deren Kristallstruktur wichtig.

Im allgemeinen sollen die festen Sorbitole folgende Eigenschaften aufweisen:

Gute Riesel- bzw. Fließfähigkeit, kein Zusammenbacken bei der Lagerung, rasche Geschmackentfaltung durch hohe Lösegeschwindigkeit, möglichst geringer Wassergehalt, gute Tablettierfähigkeit, hohe Schüttgewichte sowie eine dem jeweiligen Verwendungszweck angepasste Korngröße mit möglichst engem Korngrößenverteilungsspektrum.

Die Herstellung von Sorbitol in fester Form erfolgt entweder durch Kristallisation nahezu wasserfreier Schmelzen oder durch Sprühtrocknung von Sorbitollösungen. Ein Verfahren zur Sprühtrocknung ist aus der DE 32 45 170 A1 bekannt. Eine auf einen Feststoffgehalt von 65 bis 75 Gew.-% eingestellte Sorbitollösung wird mittels eines Zentrifugalzerstäubers in einen auf eine Temperatur von 140 bis 170 °C erwärmten trockenen zentrifugal eingeblasenen Luftstrom versprüht. Dabei wird die Menge der zugeführten Sorbitollösung und der eingeblasenen Heißluft so abgestimmt, dass das erhaltene Endprodukt nur noch einen Wassergehalt von 0,3 bis 1 % aufweist.

Der auf diese Weise hergestellte Sorbitol weist gegenüber dem durch Kristallisation aus der Schmelze erhaltenen Produkt bessere Tablettiereigenschaften auf. Die Nachteile bestehen darin, dass der nach diesem Verfahren sprühgetrocknete Sorbitol mit einer mittleren Korngröße von 0,5 mm und einem Schüttgewicht von 0,4 g/ml eine geringe Fließfähigkeit aufweist.

In der DD 252 003 B1 ist ein Verfahren beschrieben, bei dem eine 50 bis 80%ige Sorbitollösung unter ähnlichen Bedingungen (Luftstromtemperatur 120 bis 150 °C) sprühgetrocknet wird, wobei die versprühte Lösung mit in einem Luftstrom fein verteilten kristallisierten Sorbitolpartikeln vermischt wird. Das am Sprühturmaustritt anfallende Trockensorbitol wird zur weiteren Kristallisation noch 20 bis 90 min bei Temperaturen von 40 bis 80 °C behandelt und anschließend in einer Kühltrommel auf eine Temperatur von 40 °C abgekühlt. Der nachkristallisierte Sorbitol besitzt jedoch eine zu geringe Schüttdichte und ein schlechtes Rieselverhalten und ist daher für eine direkte Weiterverarbeitung ungeeignet. Zur Verbesserung dieser Eigenschaften wird daher vorgeschlagen, den nachkristallisierten Sorbitol einer zusätzlichen mechanischen Behandlung, Durchlauf durch einen Desintegrator oder durch pneumatische Förderung über eine mit Schikanen versehene Förderstrecke, zu unterziehen.

Ein Verfahren zur Herstellung von schnell löslichem Sorbitol in frei fließender Formation aus lose vereinigten Mikrokristallen mit in der Mitte offenen Kügelchen durch Sprühtrocknung ist aus der EP 0 380 219 B1 bekannt. Eine 70%ige Sorbitollösung wird auf eine Temperatur von 80 bis 85 °C erwärmt und einem Drehzerstäuber zugeführt, der bei einem Druck von 50 bis 100 MPa arbeitet. Sorbitolkeime werden in einem Schwingtrichter gehalten, durch eine Entklumpungsmaschine geleitet, dem Drehzerstäuber zugeführt und mit der Sorbitollösung vermischt. In die Entklumpungseinrichtung wird außerdem im Kreislauf geführtes Sorbitol-Feinpulver eingeleitet. Während des Zerstäubungsvorganges wird Luft mit einer Temperatur von 140 bis 160 °C in den Sprühturm eingeblasen. Der am Sprühturmaustritt anfallende mikrokristalline Sorbitol besitzt einen Feuchtigkeitsgehalt von 2 bis 10 % und wird in einem nachgeschalteten Kühlrohr abgekühlt und in einem Fließbettrockner bis auf einen Feuchtigkeitsgehalt von < 1 % getrocknet. Die den Fließbettrockner verlassenden Agglomerate müssen in einer abschließenden Sieb- oder Sichteinrichtung auf die gewünschte Korngröße zerkleinert werden. Die Nachteile dieser Produkte sind schlechtes Fließverhalten, sehr geringe Schüttgewichte und Wassergehalte, die je nach Effektivität der Nachtrocknung zwischen 0,3 bis 2 % und damit relativ hoch liegen.

Um die Eigenschaften der Sorbitole zu verbessern, ist es auch bekannt (EP 1 008 602 A1), aus der Kristallisation gewonnene, pulverförmige Sorbitole einer erneuten Aufarbeitung zu unterziehen, entweder in einem Mischer-Granulator oder in einem Zerstäuber, jeweils unter Zusatz von Wasser als Bindemittel. Diese nochmalige Aufarbeitung von in Pulverform vorliegendem Sorbitol ist mit einem hohen Aufwand und zusätzlichen Kosten verbunden.

Die bisher bekannten Verfahren zur Herstellung von sprühgetrocknetem Sorbitol haben generell den Nachteil, dass der unmittelbar am Sprühturmaustritt anfallende Sorbitol in Form von größeren Agglomeraten vorliegt, einen relativ hohen Wassergehalt von ca. 5 bis 8 % besitzt und der Kristallisationsprozess noch nicht vollständig abgeschlossen ist. Dadurch sind kostenintensive, aufwendige Nachbehandlungen des Sorbitols erforderlich.

Der Erfindung liegt die Aufgabe zugrunde, ein kostengünstiges Verfahren zur Herstellung von sprühgetrocknetem Sorbitol mit sehr guten anwendungstechnischen Eigenschaften zu schaffen, mit dem es möglich ist, bereits am Sprühturmaustritt Sorbitolpartikel mit einer kleinen Korngröße, einem niedrigen Wassergehalt und einer niedrigen Temperatur zu erhalten.

Erfindungsgemäß wird die Aufgabe durch die im Anspruch 1 angegebenen Merkmale gelöst. Geeignete Weiterbildungen der Verfahrensweise sind Gegenstand der Ansprüche 2 bis 7. Die Ansprüche 8 und 9 beziehen sich auf erfindungsgemäß hergestellte Sorbitole.

Mit den vorgeschlagenen Maßnahmen, die Sorbitollösung vor der Zuführung zu dem Zerstäuber auf eine Viskosität von 8 bis 50 mPa.s einzustellen und bei Drehzahlen des Zerstäubers von 5000 bis 15000 U/min zu versprühen, das Kreislaufkristallisat in einer definierten Menge von 3 bis 4 kg/kg Endprodukt zuzuführen und die Ablufttemperatur am Sprühturmaustritt auf einem konstanten Wert von 55 bis 57 °C zu halten, wird am Sprühturmaustritt bereits ein Produkt mit einem niedrigen Wassergehalt von kleiner 1 % und einer maximalen Korngröße von 1 mm erhalten, das zur Erzielung des fertigen Endproduktes nur noch kurzzeitig, bis maximal 6 min, auf einem Fließbett getrocknet werden muss. Bei den bisher bekannten Sprühtrocknungsverfahren beträgt der Wassergehalt der Produkte am Sprühturmaustritt noch 5 bis 8 %. Durch den niedrigen Wassergehalt von kleiner 1 % wird andererseits erreicht, dass der Kristallisationsprozess des Sorbitols trotz der geringen Verweilzeit im Sprühturm nahezu abgeschlossen werden kann. Die Einhaltung der Ablufttemperatur bei konstant 55 bis 57 °C spielt eine entscheidende Rolle. Bei höheren Ablufttemperaturen von beispielsweise 58 bis 60 °C werden bereits Agglomerate mit einem Durchmesser von 1,5 bis 2 mm erhalten, während bei geringeren Ablufttemperaturen, unterhalb von 55 °C, der Wassergehalt deutlich ansteigt.

Das am Sprühturmaustritt anfallende Produkt kann direkt in der erforderlichen Menge als Kreislaufkristallisat eingesetzt werden. Dadurch verringert sich die im nachfolgenden Fließbett zu behandelnde Menge, wodurch zusätzliche Kosteneinsparungen entstehen. Die ansonsten üblichen Verfahrensschritte einer Kühlung und mechanischen Zerkleinerung des am Sprühturmaustritt anfallenden Produktes können somit entfallen. Das am Sprühturmaustritt erhaltene Produkt entspricht in seiner Korngröße von 0,3 bis 1 mm und einer Temperatur von kleiner als 58 °C bereits annähernd dem gewünschten Endprodukt. Aufgrund der vorgeschlagenen Maßnahmen wird im Sprühturm die Bildung größerer Agglomerate gezielt vermieden, was sich auch vorteilhaft auf die sehr guten anwendungstechnischen Eigenschaften des Endproduktes auswirkt, die nachfolgend angegeben sind:

- Wassergehalt kleiner 0,2 %;
- Schüttdichte von 0,53 bis 0,62 kg/l (in Abhängigkeit von Korngrößen);
- sehr gutes Fließverhalten (Böschungswinkel kleiner 33°, Siebfraktion kleiner 0,04 mm max. 5 %, Fließgeschwindigkeit mind. 20 ml/s);
- hohe Lösegeschwindigkeiten (Lösezeit von 1g Substanz in 200 ml Wasser bei 30 °C unter Rühren max. 6 sec, spezifische Oberfläche mind. 1,4 $m^2$/g);
- gute Tablettierbarkeit (Presskraft 20 kN):

   Bruchfestigkeit: 320 bis größer 450 N (in Abhängigkeit von der Korngröße) Biegefestigkeit: 12 N/$mm^2$;

- anwendungstechnisch vorteilhafte mittlere Korngrößen von 0,12 bis 0,60 mm (enges Korngrößenspektrum, die Siebfraktion mit einer Partikeldurchmesserdifferenz von 0,2 mm beträgt mehr als 75 %).

Durch Variation der Drehzahlen des Rotationszerstäubers in dem angegebenen Bereich sowie der Viskosität wird die Tröpfchengröße der versprühten Sorbitollösung beeinflusst. Die erforderliche Viskosität der Sorbitollösung wird mittels eines Wärmetauschers durch Erwärmen auf eine Temperatur von 40 bis 95 °C eingestellt.

Zur Erzielung der gewünschten Korngrößen werden pro kg Trockensubstanz gelöst (= 1kg sprühgetrocknetes ausgekreistes Endprodukt) 3,0 bis 4,0 kg Kristallisat im Kreislauf gefahren. Die Menge des Endproduktes ergibt sich aus der Konzentration und der Einsatzmenge an Sorbitollösung.

Die zugeführte Warmluftmenge beträgt 14 bis 22 $Nm^3$ pro kg Endprodukt und richtet sich auch nach der zu verdampfenden Wassermenge.

Die Temperatur der Warmluft wird unter vorgenannten Bedingungen auf Werte zwischen 105 und 150 °C am Vorheizer geregelt, in Abhängigkeit von der kontinuierlich gemessenen Ablufttemperatur, wobei die Regelstrecke so ausgelegt ist, dass die Ablufttemperatur konstant in einem Bereich von 55 bis 57 °C gehalten wird. Der vorgesehene Temperaturbereich für die Warmluft ist deshalb relativ groß, weil verfahrenstechnisch unterschiedliche Wärmeabstrahlungen zu kompensieren sind. Das am Sprühturmaustritt anfallende Produkt wird mittels einer Förderschnecke zum Fließbett transportiert.

Die Förderleistung der Förderschnecke ist so ausgelegt, dass diese eine größere Menge transportieren kann als die Menge an Produkt, die am Sprühturmaustritt anfällt. Dadurch kann sich am Boden des Sprühturmaustritts keine Bodenschicht ausbilden und ein unerwünschtes Wachstum der Agglomerate wird so verhindert.

Die sich in der Abluft noch befindliche Menge an Sorbitolpulver wird über einen Zyklon abgeschiedenen und wieder in den Sprühturm zurückgeführt, vorzugsweise zusammen mit am Sprühturmaustritt anfallendem Sorbitol.

Das am Sprühturmaustritt mit einer relativ niedrigen Temperatur von ca. 56 °C anfallende Produkt wird mittels einer Förderschnecke zum nachgeschalteten Fließbett transportiert und auf diesem mit Warmluft (ca. 85 bis 90 °C) getrocknet und anschließend mit entfeuchteter Kaltluft (ca. 30 °C) auf eine Temperatur von 20 bis 40°C abgekühlt. Die Trocknung der noch warmen und relativ kleinen Teilchen ermöglicht, dass Wassergehalte von deutlich kleiner als 0,2 % erzielt werden. Dieser geringe Wassergehalt begünstigt - soweit erforderlich - die Nachkristallisation des sprühgetrockneten Sorbitols auf dem Fließbett und eine Vergleichmäßigung der Partikelgrößen dadurch, dass insbesondere verzweigte Agglomerate trotz der nur geringen mechanischen Belastung auf dem Fließbett an den dünnen Verbindungsstellen brechen, ohne dass feiner Abrieb entsteht. Letzteres ist für die gute Fließfähigkeit der auf diese Weise hergestellten sprühgetrockneten Sorbitole von entscheidender Bedeutung.

Aufgrund der geringen Partikelgröße von 0,3 bis 1 mm des dem Fließbett zugeführten sprühgetrockneten Sorbitols sowie dessen Wassergehalt von < 1 % werden die angestrebten Konditionierungseffekte in relativ kurzer Verweilzeit auf dem Fließbett erreicht. Während nach den bekannten Verfahren Verweilzeiten von 30 bis 90 Minuten erforderlich sind, ist nach dem erfindungsgemäßen Verfahren bereits eine Verweilzeit des sprühgetrockneten Sorbitols auf dem Fließbett von 6 Minuten ausreichend. Dadurch wird die mechanische Belastung des Produktes erheblich verringert, wobei die Siebfraktion < 0,040 mm des das Fließbett verlassenden Produktes weniger als 5 % beträgt. Damit ist das nach dem erfindungsgemäßen Verfahren hergestellte Produkt bereits ohne Siebung oder Sichtung fließfähig.

Ein weiterer Vorteil der erfindungsgemäßen Verfahrensweise besteht darin, dass durch Variation der Verfahrensparameter innerhalb der angegebenen Bereiche gezielt Sorbitole verschiedener Korngrößen, ohne gesonderte Mahl- und Siebprozesse, hergestellt werden können.

Nach dem erfindungsgemäßen Verfahren können prinzipiell alle kristallisationsfähigen Lösungen von Sorbitolen einschließlich evtl. Zugaben geringer Mengen von Süßstoffen (z.B. 0,1 % Saccharin) zu sprühgetrockneten Produkten verarbeitet werden. Vorzugsweise werden als Ausgangsprodukt 70 %ige Sorbitollösungen eingesetzt.

Beispiele

Für die nachfolgenden Beispiele 1 bis 6 wird als Ausgangsprodukt jeweils eine 70 %ige Sorbitollösung mit folgender Zusammensetzung (HPLC) eingesetzt:

$$\sum \textbf{Zuckeralkohole} \ \geq \ \textbf{DP 2 = 0,4 \%}$$

| | |
|---|---|
| Mannitol | = 0,4 % |
| Sorbitol | = 99,1 % |
| Iditol | = 0,1 % |

Viskositätsmessungen (Brookfield-Viskosimeter) der 70 %igen Sorbitollösung bei verschiedenen Temperaturen:

| | |
|---|---|
| 40 °C : 50,3 mPa.s | 70 °C : 16,1 mPa.s |
| 50 °C : 32,8 mPa.s | 80 °C : 11,9 mPa.s |
| 60 °C : 22,2 mPa.s | 90 °C : 9,0 mPa.s |

Die Sorbitollösungen werden mittels Wärmetauscher auf unterschiedliche Viskositäten eingestellt, innerhalb einer modifizierten Sprühtrocknungsanlage in unterschiedlichen Mengen dem am Kopf des Sprühturmes angeordneten Rotationszerstäuber zugeführt und versprüht. Gleichzeitig werden am Kopf des Sprühturmes die mittels Lufterhitzer auf die gewünschte Temperatur erwärmte Luft eingeblasen und die im Kreislauf zu führende sprühgetrocknete Sorbitolmenge fein verteilt. Die jeweiligen Verfahrensbedingungen sind in der nachfolgenden Tabelle 1 angegeben.

Die Abluft wird aus dem Sprühturm an dessen Bodenentleerung sowie am Übergang vom zylindrischen zum konischen Teil über einen Zyklon zur Abscheidung von Pulver geführt. Die Temperatur der aus dem Sprühturm abgeführten Abluft wird kontinuierlich gemessen und der jeweilige Messwert an eine elektronische Steuer- und Regelungsanlage übermittelt, die die Temperatur für den Lufterhitzer überwacht und regelt. Bei Abweichungen vom vorgegebenen Sollwert von 56 ± 1 °C für die Abluft wird automatisch die Lufttemperatur für die zuzuführende Warmluft verändert.

Mittels einer Förderschnecke werden die erforderliche Kreislaufkristallisatmenge in einen Zwischenbehälter und die als Endprodukt bestimmte Menge dem Fließbetttrockner zugeführt. Die entsprechende Kreislaufkristallisatmenge wird aus dem Zwischenbehälter mittels einer weiteren Förderschnecke zum Kopf des Sprühturmes transportiert.

Die auf dem Fließbett befindliche Menge an Sorbitol wird zunächst mit Warmluft von ca. 85-90 °C bis zu einem Wassergehalt von < 0,2 % getrocknet und anschließend mit entfeuchteter Kaltluft, die auf eine Temperatur von 30 bis 32 °C eingestellt wird, auf ca. 35 bis 40 °C abgekühlt.

Das im Zyklon abgeschiedene Pulver wird mittels Gebläse abgezogen und mit dem im Kreislauf geführten Kristallisat vereinigt.

Die Verfahrensparameter (siehe Tabelle 1) für die Beispiele 1 bis 6 wurden gezielt verändert und belegen, dass sich innerhalb der angegebenen Bereichsgrenzen auch Sorbitole mit unterschiedlichen Korngrößen herstellen lassen.

Beispiel 1

Herstellung von Sorbitol TFF mit einer durchschnittlichen Korngröße von 0,12 bis 0,15 mm.

Beispiel 2

Herstellung von Sorbitol TF mit einer durchschnittlichen Korngröße von 0,16 bis 0,20 mm.

Beispiel 3

Herstellung von Sorbitol T mit einer durchschnittlichen Korngröße von 0,21 bis 0,27 mm.

Beispiel 4

Herstellung von Sorbitol TG mit einer durchschnittlichen Korngröße von 0,35 bis 0,45 mm.

Beispiel 5

Herstellung von Sorbitol TGG mit einer durchschnittlichen Korngröße von 0,50 bis 0,60 mm.

Beispiel 6

Herstellung von Sorbitol TS mit einer durchschnittlichen Korngröße von 0,21 bis 0,27 mm, wobei dem Ausgangsprodukt zur Anhebung der Süßkraft 0,1 % Saccharin (bezogen auf Trockensubstanz) zugesetzt wurde.

Eigenschaften der sprühgetrockneten Produkte:

An den nach den Beispielen 1 bis 6 hergestellten Sorbitol-Endprodukten wurden folgende Eigenschaftswerte bestimmt:

- Wassergehalt nach Karl Fischer;
- Schüttdichte gemäß DIN ISO 3944;
- Böschungswinkel $\alpha$ : $tg\alpha$ = Schütthöhe/Radius;
- Fließgeschwindigkeit: ERWEKA-Auslauftrichter GD1;
- Lösezeit: Lösezeit von 1g Substanz in 200 ml dest. Wasser bei 30 °C unter Rühren;
- Korngröße: (Siebanalyse) nach Alpine Maschensiebverfahren;
- Spezifische Oberfläche: FlowSorb **II** 2300 (Micrometrics, Norcoss, Georgia, USA), Meßgas 70 % He/30 % $N_2$

Die in Tabelle 1 angegebenen Eigenschaftswerte des am Sprühturmaustritt erhaltenen Produktes wurden ebenfalls nach vorgenannten Methoden bestimmt.

Aus den Sorbitol-Endprodukten gemäß den Beispielen 1 bis 6 wurden Tabletten unter folgenden Bedingungen hergestellt:

Rundlauftablettenpresse Korsch (Berlin); Stempelpaar Ø 10 mm; Tablettengewicht 500 mg; Zusatz von 1 % Mg-Stearat; Presskraft: 20 kN.

An den Tabletten wurden als Eigenschaftswerte die Bruchfestigkeit (Schleuninger 6D-Bruchfestigkeitstester) und die Biegefestigkeit (ERWEKA - Härtetester) bestimmt. Die erzielten Ergebnisse (Tablettenstabilität) sind in der Tabelle 2 angegeben.

Tabelle 1

| Produkt<br>gewünschte Korngröße Ø in mm | | | Sorbitol TFF<br>0,12 - 0,15 | Sorbitol TF<br>0,16 - 0,20 | Sorbitol T<br>0,21 - 0,27 | Sorbitol TG<br>0,35 - 0,45 | Sorbitol TGG<br>0,55 - 0,65 | Sorbitol TS*<br>0,21 - 0,27 |
|---|---|---|---|---|---|---|---|---|
| Beispiel-Nr. | | | 1 | 2 | 3 | 4 | 5 | 6 |
| Sorbitollösung | Konzentration | (%) | 70 | 70 | 70 | 70 | 70 | 70 |
| | Temperatur | (°C) | 92 | 90 | 87 | 50 | 42 | 87 |
| | Viskosität | (mPa.s) | 8,5 | 9 | 10 | 33 | 48 | 10 |
| | Menge | (kg/h) | 1250 | 1280 | 1300 | 1450 | 1450 | 1300 |
| Warmluft | Temperatur | (%) | 128 | 120 | 113 | 131 | 140 | 118 |
| | Menge | (Nm$^3$/h) | 12250 | 14350 | 16400 | 20300 | 21200 | 16400 |
| | -pro kg Endprordukt | (Nm$^3$) | 14 | 16 | 18 | 20 | 21 | 18 |
| Kreislaufkristallisat | Menge | (kg/h) | 2625 | 2970 | 3275 | 3860 | 4060 | 3275 |
| | -pro kg Endprodukt | (kg) | 3,0 | 3,3 | 3,6 | 3,8 | 4,0 | 3,6 |
| Rotationszerstäuber | Drehzahl | (U/min) | 15000 | 14000 | 12000 | 5500 | 5000 | 12000 |
| Abluft | Temperatur | (°C) | 56,0 | 55,7 | 56,3 | 56,8 | 57,0 | 56,6 |
| Produktaustritt Sprühturm | max. Korngröpe | (mm) | 0,3 | 0,4 | 0,5 | 0,8 | 1,0 | 0,5 |
| | Wassergehalt | (%) | 0,68 | 0,83 | 0,82 | 0,95 | 0,95 | 0,74 |
| | Temperatur | (°C) | 56,2 | 56,1 | 56,9 | 56,8 | 56,9 | 55,9 |
| Produktaustritt Fließbett | Menge | (kg/h) | 875 | 900 | 910 | 1015 | 1015 | 910 |
| | Wassergehalt | (%) | 0,11 | 0,13 | 0,15 | 0,15 | 0,17 | 0,14 |
| * Zusatz von 0,1 % Saccharin | | | | | | | | |

Tabelle 2

| Produkt | | Sorbitol TFF | Sorbitol TF | Sorbitol T | Sorbitol TG | Sorbitol TGG | Sorbitol TS* |
|---|---|---|---|---|---|---|---|
| gewünschte Korngröße Ø in mm | | 0,12 - 0,15 | 0,16 - 0,20 | 0,21 - 0,27 | 0,35 - 0,45 | 0,50 - 0,60 | 0,21 - 0,27 |
| Beispiel-Nr. | | 1 | 2 | 3 | 4 | 5 | 6 |
| Wassergehalt | (%) | 0,11 | 0,13 | 0,15 | 0,15 | 0,17 | 0,14 |
| Schüttdichte | (kg/l) | 0,61 | 0,6 | 0,58 | 0,56 | 0,54 | 0,57 |
| Böschungswinkel | (Grad) | 32,8 | 32,6 | 32,2 | 31,8 | 31,8 | 31,9 |
| Fließgeschwindigkeit | (ml/s) | 32 | 40 | 23 | 38 | 34 | 25 |
| Lösezeit | (s) | 3,5 | 4 | 5 | 5 | 5 | 5 |
| Spezifische Oberfläche | (m$^2$/g) | 1,6 | 1,5 | 1,4 | 1,4 | 1,4 | 1,4 |
| Tablettenstabilität | | | | | | | |
| Bruchfestigkeit | (N) | >450 | >450 | 420 | 350 | 320 | nicht zur Tablettierung vorgesehen |
| Biegefestigkeit | (N/mm$^2$) | 11,9 | 12,2 | 12 | 11 | 10,5 | |
| Siebanalyse | | | | | | | |
| < 0,04 | mm | 3 | 2 | 1 | | | |
| 0,04-0,1 | mm | 28 | 5 | 2 | 1 | | 3 |
| 0,1-0,2 | mm | 54 | 36 | 18 | 2 | 2 | 25 |
| 0,2-0,3 | mm | 15 | 45 | 59 | 6 | 2 | 60 |
| 0,3-0,4 | mm | | 2 | 17 | 28 | 5 | 10 |
| 0,4-0,5 | mm | | | 3 | 49 | 10 | 2 |
| 0,5-0,8 | mm | | | | 13 | 76 | |
| > 0,8 | mm | | | | 1 | 5 | |
| Ø Korngröße | mm | 0,13 | 0,18 | 0,25 | 0,43 | 0,56 | 0,23 |

* Zusatz von 0,1 % Saccharin

**Patentansprüche**

1. Verfahren zur Herstellung von sprühgetrocknetem Sorbitol durch Versprühen einer 65 bis 75%igen Sorbitollösung mittels eines Rotationszerstäubers in einem Sprühturm im Gleichstrom mit feinverteiltem Sorbitol-Kreislaufkristallisat unter Zuführung von Warmluft und einem nachgeschalteten Fließbett zur weiteren Trocknung der Sorbltolpartikel, **dadurch gekennzeichnet, dass** die Sorbitollösung vor der Zuführung zu dem Zerstäuber auf eine Viskosität von 8 bis 50 mPa.s eingestellt wird, der Zerstäuber mit einer Drehzahl von 5000 bis 15000 U/min betrieben wird, Kreislaufkristallisat in einer Menge von 3 bis 4 kg/kg Endprodukt zugeführt wird und am Sprühturmaustritt die Temperatur der Abluft kontinuierlich gemessen und über eine Regelstrecke die Temperatur der zugeführten Warmluft in einem Bereich von 105 bis 150 °C verändert wird, derart, dass die Ablufttemperatur auf einem konstanten Wert von 55 bis 57 °C gehalten wird, wobei Warmluft in einer Menge von 14 bis 22 m$^3$/kg Endprodukt zugeführt wird, und abschließend am Sprühturmaustritt anfallender Sorbitol direkt dem nachgeschalteten Fließbett zugeführt, mittels Warmluft mit einer Temperatur von 85 bis 90 °C getrocknet und abschließend mit entfeuchteter Kaltluft auf eine Temperatur von 20 bis 40 °C abgekühlt wird.

2. Verfahren nach Anspruch 1, **dadurch gekennzeichnet, dass** die Zeitdauer der Nachbehandlung auf dem Fließbett maximal 6 min beträgt.

3. Verfahren nach einem der Ansprüche 1 oder 2, **dadurch gekennzeichnet, dass** das Sorbitol-Kreislaufkristallisat aus von am Sprühturmaustritt anfallendem Sorbitol und aus der Abluft abgeschiedenem Sorbitol gebildet wird.

**4.** Verfahren nach einem der Ansprüche 1 bis 3, **dadurch gekennzeichnet, dass** am Sprühturmaustritt anfallender Sorbitol kontinuierlich, ohne Bildung einer Bodenschicht im Sprühturm, mittels einer Fördereinrichtung zum nachgeschalteten Fließbett transportiert wird.

**5.** Verfahren nach einem der Ansprüche 1 bis 4, **dadurch gekennzeichnet, dass** bereits am Sprühturmaustritt Sorbitole mit einer Korngröße von 0,3 bis 1mm mit einem Wassergehalt von unter 1 % und einer Temperatur von kleiner als 58 °C erhalten werden.

**6.** Verfahren nach einem der Ansprüche 1 bis 5, **dadurch gekennzeichnet, dass** nach der Fließbettbehandlung Sorbitol mit einem Wassergehalt von kleiner als 0,2 % und einer Siebfraktion < 0,04 mm von unter 5 % erhalten wird.

**7.** Verfahren nach einem der Ansprüche 1 bis 6, dadurch gekennzeichet, dass als Endprodukt Sorbitole mit einem engen Korngrößenspektrum erhalten werden, wobei die jeweilige Siebfraktion mit einer Partikeldurchmesserdifferenz von 0,2 mm mindestens 75 % beträgt.

**8.** Sorbitol, hergestellt nach mindestens einem der vorhergehenden Ansprüche, mit folgenden Eigenschaften:

- einer durchschnittlichen Korngröße von 0,12 bis 0,60 mm,
- einer Schüttdichte von 0,53 bis 0,61 kg/l,
- einer Fließgeschwindigkeit von mindestens 20 ml/s,
- einem Böschungswinkel von maximal 33°,
- einer spezifischen Oberfläche von mindestens 1,4 m$^2$/g,
- einer Lösezeit (1g Sorbitol in 200 ml Wasser bei 30 °C unter Rühren) von maximal 5 s.

**9.** Sorbitol nach Anspruch 8, **dadurch gekennzeichnet, dass** dieser eine durchschnittliche Korngrößen von 0,12 bis 0,45 mm aufweist, mit einem Anteil von mehr als 75 % der Siebfraktion mit einer Maschenweitendifferenz von 0,2 mm.

**Claims**

**1.** Method for the preparation of spray-dried sorbitol by spraying a 65 to 75% sorbitol solution by means of a rotary atomizer in a spray tower concurrently with circulated fine sorbitol crystals while supplying hot air and a downstream fluidized bed for the further drying of the sorbitol particles, **characterized in that** the sorbitol solution is set to a viscosity of 8 to 50 mPa s before it is supplied to the atomizer, the atomizer is operated at a rotational speed of 5000 to 15000 rpm, the circulated crystals are supplied at an amount of 3 to 4 kg/kg of end product and the temperature of the exhaust air is continuously measured at the spray tower outlet and the temperature of the hot air supplied is changed via a control system over a range of 105 to 150°C, such that the exhaust air temperature is maintained at a constant value of 55 up to 57°C, wherein hot air is supplied at an amount of 14 to 22 m$^3$/kg of final product, and finally sorbitol accruing at the spray tower outlet is supplied directly to the downstream fluidized bed, dried by means of hot air having a temperature of 85 to 90°C and finally cooled by dehumidified cold air to a temperature of 20 to 40°C.

**2.** Method according to claim 1, **characterized in that** the duration of the posttreatment in the fluidized bed is a maximum of 6 min.

**3.** Method according to one of the claims 1 to 2, **characterized in that** the circulated sorbitol crystals are formed from the sorbitol accruing at the spray tower outlet and the sorbitol deposited from the exhaust air.

**4.** Method according to one of the claims 1 to 3, **characterized in that** sorbitol accruing at the spray tower outlet is continuously transported by means of a conveyor to the downstream fluidized bed without forming a ground layer in the spray tower.

**5.** Method according to one of the claims 1 to 4, **characterized in that** sorbitols with a particle size of 0.3 to 1 mm, a water content of less than 1% and a temperature of less than 58°C are already obtained at the spray tower outlet.

**6.** Method according to one of the claims 1 to 5, **characterized in that** sorbitol having a water content of less than 0.2% and a sieve fraction <0.04 mm of under 5% is obtained after the fluidized bed treatment.

**7.** Method according to one of the claims 1 to 6, **characterized in that** sorbitols are obtained as an end product with a narrow particle size spectrum, wherein the respective sieve fraction with a particle diameter difference of 0.2 mm amounts to at least 75%.

8. Sorbitol prepared according to at least one of the preceding claims having the following properties:

    - an average particle size of 0.12 to 0.60 mm,
    - a bulk density of 0.53 to 0.61 kg/l,
    - a flow rate of at least 20 ml/s,
    - an angle of repose of a maximum of 33°,
    - a specific surface area of at least 1.4 $m^2$/g and
    - a dissolution time of a maximum of 5 s (1 g sorbitol in 200 ml water at 30°C with stirring).

9. Sorbitol according to claim 8, **characterized in that** this has an average particle size from 0.12 to 0.45 mm, with a proportion of more than 75% of the sieve fraction having a mesh width difference of 0.2 mm.

**Revendications**

1. Procédé pour la fabrication de sorbitol séché par atomisation en pulvérisant une solution contenant de 65 à 75 % de sorbitol au moyen d'un atomiseur rotatif dans une tour d'atomisation dans un courant parallèle au produit de cristallisation du circuit de sorbitol, finement réparti, en rajoutant de l'air chaud et un lit fluidisé, placé à la suite, afin de continuer à sécher les particules de sorbitol, **caractérisé en ce que** la solution de sorbitol est portée à une viscosité comprise entre 8 et 50 mPa·s avant d'être introduite dans l'atomiseur, l'atomiseur est actionné à une vitesse de rotation comprise entre 5 000 et 15 000 tr/min le produit de cristallisation du circuit est rajouté dans une quantité comprise entre 3 et 4 kg/kg de produit final et la température de l'air évacué est mesurée en continu à la sortie de la tour d'atomisation et la température de l'air chaud amené est modifiée par le biais d'un circuit de régulation dans une plage comprise entre 105 et 150 °C de sorte que la température de l'air évacué est maintenue à une valeur constante comprise entre 55 et 57 °C, de l'air chaud étant rajouté dans une quantité comprise entre 14 et 22 $m^3$/kg de produit final, et pour finir, le sorbitol produit à la sortie de la tour d'atomisation est introduit directement dans le lit fluidisé, placé à la suite, séché au moyen d'air chaud à une température comprise entre 85 et 90 °C pour être finalement refroidi à l'aide d'air froid déshydraté à une température comprise entre 20 et 40 °C.

2. Procédé selon la revendication 1, **caractérisé en ce que** la durée du traitement postérieur sur le lit fluidisé est de 6 min au maximum.

3. Procédé selon l'une quelconque des revendications 1 ou 2, **caractérisé en ce que** le produit de cristallisation du circuit de sorbitol est formé par le sorbitol produit à la sortie de la tour d'atomisation et par le sorbitol séparé de l'air évacué.

4. Procédé selon l'une quelconque des revendications 1 à 3, **caractérisé en ce que** le sorbitol produit à la sortie de la tour d'atomisation est transporté en continu vers le lit fluidisé, placé à la suite, au moyen d'un dispositif de transport sans former de couche au fond de la tour d'atomisation.

5. Procédé selon l'une quelconque des revendications 1 à 4, **caractérisé en ce que**, dès leur sortie de la tour d'atomisation, on obtient des sorbitols d'une granulométrie comprise entre 0,3 et 1 mm, d'une teneur en eau inférieure à 1 % et d'une température inférieure à 58 °C.

6. Procédé selon l'une quelconque des revendications 1 à 5, **caractérisé en ce qu'**au terme du traitement en lit fluidisé, on obtient un sorbitol d'une teneur en eau inférieure à 0,2 % et d'une fraction tamisée de < 0,04 mm inférieure à 5 %.

7. Procédé selon l'une quelconque des revendications 1 à 6, **caractérisé en ce qu'**on obtient en produit final des sorbitols d'un spectre de granulométrie étroit dont chaque fraction tamisée, présentant une différence de diamètre de particules de 0,2 mm, est d'au moins 75 %.

8. Sorbitol fabriqué d'après au moins l'une des revendications précédentes, présentant les propriétés suivantes :

    - granulométrie moyenne comprise entre 0,12 et 0,60 mm,
    - densité en vrac comprise entre 0,53 et 0,61 kg/l,

- vitesse d'écoulement d'au moins 20 ml/s,
- angle d'éboulement de 33° maximum,
- surface spécifique d'au moins 1.4 m$^2$/g et
- temps de dissolution (1 g de sorbitol dans 200 ml d'eau à 30 °C en remuant) de 5 s maximum.

9. Sorbitol selon la revendication 8, **caractérisé en ce que** celui-ci présente une granulométrie comprise entre 0,12 et 0,45 mm comportant une part de fraction tamisée dont la différence de largeur de maille est de 0,2 mm supérieure à 75 %.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3245170 A1
- DD 252003 B1

- EP 0380219 B1
- EP 1008602 A1